# EUROPEAN PATENT APPLICATION

(11) **EP 2 261 205 A1**
(43) Date of publication of application: **15.12.2010**
(21) Application number: 09726472.5
(22) Date of filing: 26.03.2009
(51) Int. Cl.: C07D 207/09, C07D 207/12, C07B 61/00

(54) **METHOD OF PRODUCING (S)-3-(1-CYANO-1,1-DIPHENYLMETHYL)-PYRROLIDINE**

(30) Priority: 02.04.2008 JP 2008096393
(71) Applicant: Kaneka Corporation, Kita-ku Osaka-shi, Osaka 530-8288 (JP)
(72) Inventor: NISHIYAMA, Akira, Takasago-shi Hyogo 676-8688 (JP); OHNUKI, Masatoshi, Takasago-shi Hyogo 676-8688 (JP)
(74) Representative: Hart-Davis, Jason
(86) International application number: PCT/JP2009/056065
(87) International publication number: WO 2009/122997

(57) **Abstract**

The present application relates to a method of (S)-3-(1-cyano-1,1-diphenylmethyl)-pyrrolidine by reacting an (S)-1-protected-3-(sulfonyloxy)-pyrrolidine having an easily deprotectable protecting group with diphenylacetonitrile in the presence of a base to obtain an (S)-1-protected-3-(1-cyano-1,1-diphenylmethyl)-pyrrolidine and then deprotecting the obtained compound under a mild condition. According to the method of the present invention, it is possible to efficiently produce (S)-3-(1-cyano-1,1-diphenylmethyl)-pyrrolidine, which is an important intermediate in the process of producing a muscarinic receptor antagonist such as darifenacin.

## Description

### TECHNICAL FIELD

The present invention relates to a method of (S)-3-(1-cyano-1,1-diphenylmethyl)-pyrrolidine and the important intermediates thereof. The (S)-3-(1-cyano-1,1-diphenylmethyl)-pyrrolidine is an important intermediate in the process of producing a muscarinic receptor antagonist such as darifenacin described in, for example, WO2003/0805

### BACKGROUND WART

As the method of producing (S)-3-(1-cyano-1,1-diphenylmethyl)-pyrrolidine, the following methods have been known:
1) a production method in which (S)-1-benzyl-3-(1-cyano-1,1-diphenylmethyl)-pyrrolidine is hydrogenated in the presence of a palladium catalyst (Patent Document 1); and
2) a production method in which (S)-1-(4-methylbenzenesulfonyl)-3-(1-cyano-1,1-diphenylmethyl)-pyrrolidine is heated to reflux in the presence of phenol and hydrobromic acid (Patent Document 2).

Patent Document 1: JP10-7652A
Patent Document 2: WO2003/080599

### DISCLOSURE OF THE INVENTION

### PROBLEMS TO BE SOLVED BY THE INVENTION

By the above conventional method 1), it is difficult to obtain (S)-1-benzyl-3-(1-cyano-1,1-diphenylmethyl)-pyrrolidine with high purity. It is therefore necessary to purify the product by silica gel column chromatography after debenzylation reaction, and the method is not suitable for industrial production. In addition, the above conventional method 2) is not preferable, since the method requires very high temperature for the reaction.

### MEANS FOR SOLVING THE PROBLEMS

The present inventors made various investigations; as a result, the inventors developed a method of producing (S) -1-protected-3- (1-cyano-1,1-diphenylmethyl)-pyrrolidine having an easily deprotectable protecting group under a milder condition than conventional methods.

The present invention relates to an (S)-1-protected-3-(1-cyano- 1,1-diphenylmethyl)-pyrrolidine represented by the following formula (1): 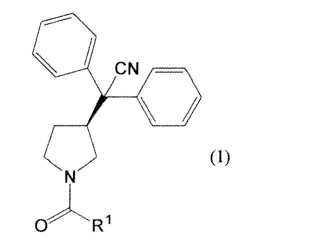 wherein, R¹ is a methyl group, a phenyl group, a tert-butoxy group or a benzyloxy group.

In addition, the present invention relates to a method of producing an (S)-1-protected-3-(1-cyano-1,1-diphenylmethyl)-pyrrolidine,
wherein the (S)-1-protected-3-(1-cyano-1,1-diphenylmethyl)-pyrrolidine is represented by the following formula (4): 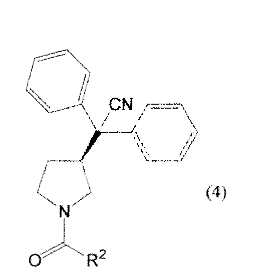 wherein, R² is a hydrogen atom, an optionally substituted alkyl group having 1 to 20 carbon atoms, an optionally substituted alkenyl group having 2 to 20 carbon atoms, an optionally substituted aralkyl group having 7 to 20 carbon atoms, an optionally substituted aryl group having 6 to 20 carbon atoms, an optionally substituted alkyloxy group having 1 to 20 carbon atoms, an optionally substituted alkenyloxy group having 2 to 20 carbon atoms, an optionally substituted aralkyloxy group having 7 to 20 carbon atoms, or an optionally substituted aryloxy group having 6 to 20 carbon atoms,
comprising a step of reacting an (S)-1-protected-3-(sulfonyloxy)-pyrrolidine represented by the following formula (2): 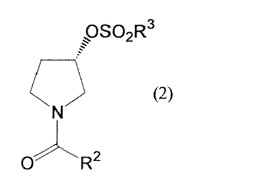 wherein, R² is the same as the above; R³ is an optionally substituted alkyl group having 1 to 20 carbon atoms, or an optionally substituted aryl group having 6 to 20 carbon atoms,
with diphenylacetonitrile represented by the following formula (3) : 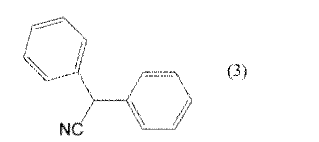 in the presence of a base.

The present invention further relates to a method of producing an (S)-1-protected-3-pyrrolidinol,
wherein the (S)-1-protected-3-pyrrolidinol is represented by the following formula (8): 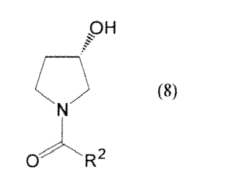 wherein R² means as the above,
comprising steps of
reducing (S)-4-chloro-3-hydroxybutyronitrile represented by the following formula (6): 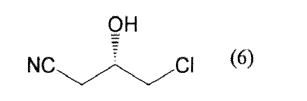 to obtain (S)-3-pyrrolidinol represented by the following formula (7): 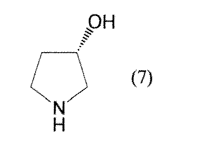 and then protecting the amino group without isolating the compound (7).

### EFFECT OF THE INTENTION

According to the present invention, it is possible to easily produce an (S)-1-protected-3-(1-cyano-1,1-diphenylmethyl)-pyrrolidine having an easily deprotectable protecting group under a milder condition than conventional methods; therefore, it is possible to produce (S)-3-(1-cyano-1,1-diphenylmethyl)-pyrrolidine more efficiently than conventional methods.

### MODE FOR CARRYING OUT THE INVENTION

Hereinafter, the present invention is described in detail.

The present invention is represented by a figure as follows.

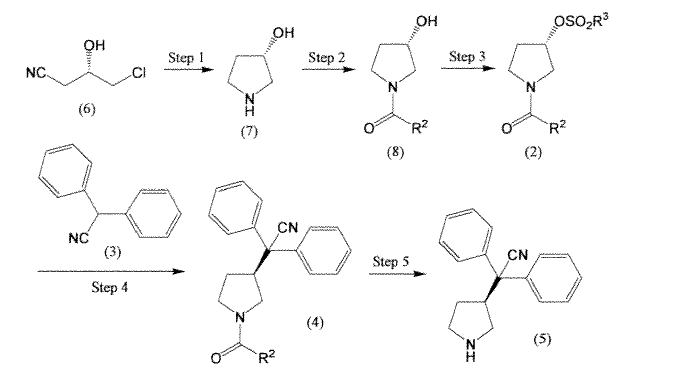

Hereinafter, each step is described in order. Unless otherwise noted, each step is independent each other and it is not necessarily the case that the target compound is obtained by carrying out all the steps in the present invention.

### Step 1

In the step 1, (S)-3-pyrrolidinol represented by the formula (7) : 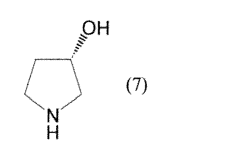 by reducing (S)-4-chloro-3-hydroxybutyronitrile represented by the formula (6).

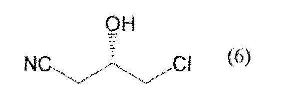

(S)-4-chloro-3-hydroxybutyronitrile represented by the formula (6) can be easily produced by the methods described in, for example, JP2-85249A and JP5-69818B.

The condition of the reduction reaction of the step 1 is briefly described hereinafter. The detail condition is described in JP2-85249A.

As the reduction reaction, a catalytic reduction method using a metal catalyst is preferable.

As the metal catalyst, Raney metal or alloy, rhodium catalyst, palladium catalyst, or platinum catalyst is exemplified. Among them, Raney nickel, Raney cobalt, Raney nickel - chrome, nickel boride, rhodium / alumina, rhodium / carbon, palladium / alumina, palladium / carbon, platinum oxide or platinum / carbon are preferable, and palladium / carbon is more preferable. A metal catalyst may be used by it self or with others.

The use amount of the metal catalyst is preferably 0.001 to 10 times by weight, and more preferably 0.01 to 1 time by weight, relative to the compound (6).

The solvent used in the step 1 is not limited as long as it is used for general catalytic reduction method, and is preferably water, methanol, ethanol, n-propanol, isopropanol, n-butanol, ethylene glycol, acetic acid, 1,4-dioxane, toluene or ethyl acetate, and more preferably methanol or ethanol. A solvent may be used by it self, or plural solvents may be mixed to be used. When mixed solvent is used, the mixing ratio is not limited.

The use amount of the solvent is preferably not more than 50 times by weight, and more preferably not more 20 times by weight, relative to the compound (6), since too much solvent is not preferable in terms of cost and post-processing.

An acid may be further added for accelerating the reduction reaction. As the acid, hydrochloric acid, hydrobromic acid, sulfuric acid, phosphoric acid, methanesulfonic acid and acetic acid are exemplified, and hydrochloric acid is preferable.

The use amount of the acid is preferably 0.1 to 10 times by mole, and more preferably 0.5 to 3 times by mole, relative to the compound (6).

The reaction product of the step 1 is mainly a primary amine which is the intermediate in the reduction reaction in some cases or the (S)-3-pyrrolidinol represented by the formula (7) in other cases depending on the used metal catalyst, reaction temperature and reaction time. Therefore, a base may be further added in order to complete the cyclization.

As such a base, lithium hydroxide, sodium hydroxide, potassium hydroxide, cesium hydroxide, magnesium hydroxide, lithium carbonate, sodium carbonate, potassium carbonate, sodium hydrogencarbonate, potassium hydrogencarbonate, trisodium phosphate, tripotassium phosphate, triethylamine, diisopropylethylamine and pyridine are exemplified, and sodium hydroxide, potassium hydroxide, sodium carbonate, potassium carbonate, sodium hydrogencarbonate, potassium hydrogencarbonate and triethylamine are preferable, and triethylamine is more preferable.

The use amount of the base is preferably 0.1 to 10 times by mole, and more preferably 0.5 to 3 times by mole, relative to the compound (6).

The pressure of hydrogen added when the reduction reaction is carried out is preferably 0.5 to 100 atm, and more preferably 1 to 10 atm.

The reaction temperature of the step 1 may be appropriately set depending on the kind and amount of the used catalyst and the hydrogen pressure, and is preferably 0 to 100°C, and more preferably 10 to 60°C.

The reaction time of the step 1 may be also appropriately set depending on the kind and amount of the used catalyst, the hydrogen pressure and the reaction temperature, and is preferably 1 to 100 hours, and more preferably 5 to 40 hours.

After the reduction reaction is completed, the reaction mixture may be directly subject to the next step or the filtrate obtained by filtration of the reaction mixture under reduced pressure and the like to remove the metal catalyst may be subject to the next step.

### Step 2

In the step 2, (S)-1-protected-3-pyrrolidinol represented by the following formula (8): 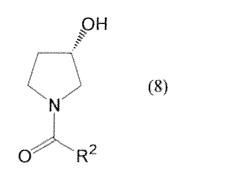 is produced by protecting the amino group without isolating (S)-3-pyrrolidinol represented the formula (7).

In the above formula, R² is a hydrogen atom, an optionally substituted alkyl group having 1 to 20 carbon atoms, an optionally substituted alkenyl group having 2 to 20 carbon atoms, an optionally substituted aralkyl group having 7 to 20 carbon atoms, an optionally substituted aryl group having 6 to 20 carbon atoms, an optionally substituted alkyloxy group having 1 to 20 carbon atoms, an optionally substituted alkenyloxy group having 2 to 20 carbon atoms, an optionally substituted aralkyloxy group having 7 to 20 carbon atoms, or an optionally substituted aryloxy group having 6 to 20 carbon atoms.

The alkyl group having 1 to 20 carbon atoms is exemplified by, for example, a methyl group, an ethyl group, an n-propyl group, an isopropyl group, an n-butyl group or a tert-butyl group. The alkenyl group having 2 to 20 carbon atoms is exemplified by, for example, a vinyl group, an allyl group or a methallyl group. The aralkyl group having 7 to 20 carbon atoms is exemplified by, for example, a benzyl group, a 1-phenethyl group or a (2,3-dihydrobenzofuran-5- yl)methyl group. The aryl group having 6 to 20 carbon atoms is exemplified by, for example, a phenyl group, a naphthyl group or a biphenyl group. The alkyloxy group having 1 to 20 carbon atoms is exemplified by, for example, a methoxy group, an ethoxy group, an isopropoxy group or a tert-butoxy group. The alkenyloxy group having 2 to 20 carbon atoms is exemplified by, for example, a vinyloxy group, an allyloxy group or a methallyloxy group. The aralkyloxy group having 7 to 20 carbon atoms is exemplified by, for example, a benzyloxy group or a 1-phenethyloxy group. The aryloxy group having 6 to 20 carbon atoms is exemplified by, for example, a phenoxy group or a biphenyloxy group.

The substituent is exemplified by, for example, halogen atoms such as a fluorine atom, a chlorine atom, a bromine atom and an iodine atom; a nitro group; a nitroso group; a cyano group; an amino group; a hydroxyamino group; an alkylamino group having 1 to 12 carbon atoms; a dialkyl amino group having 1 to 12 carbon atoms; an aralkyl amino group having 7 to 12 carbon atoms; a diaralkyl amino group having 7 to 12 carbon atoms; an alkylsulfonylamino group having 1 to 12 carbon atoms; a sulfonate group; a sulfonamide group; an azido group; a trifluoromethyl group; a carboxy group; an acyl group having 1 to 12 carbon atoms; an aroyl group having 7 to 12 carbon atoms; a hydroxy group; an alkyloxy group having 1 to 12 carbon atoms; an aralkyloxy group having 7 to 12 carbon atoms; an aryloxy group having 6 to 12 carbon atoms; an acyloxy group having 1 to 12 carbon atoms; an aroyloxy group having 7 to 12 carbon atoms; a silyloxy group having 3 to 12 carbon atoms; an alkylsulfonyloxy having 1 to 12 carbon atoms; or an alkylthio group having 1 to 12 carbon atoms.

R² is preferably a hydrogen atom, a methyl group, an ethyl group, an n-propyl group, a methoxymethyl group, a chloromethyl group, a trifluoromethyl group, a (2,3-dihydrobenzofuran-5-yl)methyl group, a phenyl group, a 4-methylphenyl group, a 4-chlorophenyl group, a 4-nitrophenyl group, a methoxy group, an ethoxy group, an isopropoxy group, a tert-butoxy group, an allyloxy group, a benzyloxy group or a phenoxy group, and more preferably a methyl group, a phenyl group, a tert-butoxy group or a benzyloxy group, especially preferably a tert-butoxy group.

When (S)-3-pyrrolidinol represented by the formula (7) is extracted using a general organic solvent, a large amount of an organic solvent is needed and the efficiency is poor, since the water solubility thereof is very high. Accordingly, in JP2-85249A, (S)-3-pyrrolidinol is isolated by concentrating the reaction mixture after the reduction reaction under reduced pressure, adding methanol and sodium hydroxide to the residue, removing the precipitated sodium chloride by filtration under reduced pressure, concentrating the filtrate under reduced pressure, and distilling the obtained residue under reduced pressure. However, the method requires many cumbersome procedures such as filtration, concentration and distillation, and has various problems for industrial production.

The present inventors found that it is possible to extract and isolate (S)-1-protected-3-pyrrolidinol using a small amount of an organic solvent by protecting the amino group without isolating (S)-3-pyrrolidinol as the step 2 and to produce the (S)-1-protected-3-pyrrolidinol.

The amino group is preferably protected with an acyl group or a carbamate group, and can be protected by adding an acylating reagent or a carbamating reagent into the reaction mixture obtained by the step 1.

The acylating reagent is exemplified by, for example, esters such as methyl formate and methyl trifluoroacetate; acyl halides such as acetyl chloride, acetyl bromide, propionyl chloride, butanoyl chloride, (2,3-dihydrobenzofuran-5-yl)acetyl chloride, benzoyl chloride, 4-methylbenzoyl chloride, 4-chlorobenzoyl chloride, 4-nitrobenzoyl chloride; anhydrides such as acetic anhydride and trifluoroacetic anhydride.

The carbamating reagent is exemplified by, for example, chlorides such as methoxycarbonyl chloride, ethoxycarbonyl chloride, isopropoxycarbonyl chloride, allyloxycarbonyl chloride, benzyloxycarbonyl chloride, phenyloxycarbonyl chloride; and anhydrides such as di-tert-butyl dicarbonate.

Among the above, acetyl chloride, acetic anhydride, benzoyl chloride, benzyloxycarbonyl chloride and di-tert-butyl dicarbonate are preferable, and di-tert-butyl dicarbonate is more preferable.

The use amount of the acylating reagent or carbamating reagent is preferably 1 to 10 times by mole, and more preferably 1 to 3 times by mole, relative to (S)-3-pyrrolidinol.

A base may be further added during the above protection in order to neutralize the generated acid as by-product and accelerate the reaction.

The base is exemplified by, for example, metal carbonates such as lithium carbonate, sodium carbonate, potassium carbonate, magnesium carbonate and calcium carbonate; metal hydrogencarbonates such as lithium hydrogencarbonate, sodium hydrogencarbonate, potassium hydrogencarbonate and cesium hydrogencarbonate; metal hydroxides such as lithium hydroxide, sodium hydroxide and potassium hydroxide; tertiary amines such as triethylamine, ethyldiisopropylamine and pyridine. Among them, sodium carbonate, potassium carbonate, sodium hydrogencarbonate, potassium hydrogencarbonate, sodium hydroxide, potassium hydroxide, triethylamine and pyridine are preferable, and triethylamine is more preferable.

The use amount of the base is preferably 0.5 to 20 times by mole, and more preferably 0.5 to 5 times by mole, relative to (S)-3-pyrrolidinol.

A reaction solvent is not particularly needed in the step 2, but may be used when the reaction is accelerated by the addition of a solvent.

Such a reaction solvent is not limited as long as the solvent has harmful effects on the reaction, and, for example, water; alcohol solvents such as methanol, ethanol, n-propanol, isopropanol, n-butanol, tert-butanol and ethylene glycol; ether solvents such as tetrahydrofuran, diethyl ether, 1,4-dioxane and ethylene glycol dimethyl ether; aromatic hydrocarbon solvents such as benzene and toluene; aliphatic hydrocarbon solvents such as pentane, hexane, heptane and methylcyclohexane; halogenated solvents such as carbon tetrachloride, chloroform, methylene chloride, 1,2-dichloroethane and chlorobenzene; ester solvents such as ethyl acetate, isopropyl acetate and tert-butyl acetate; sulfoxide solvents such as dimethylsulfoxide; amide solvents such as N,N-dimethylformamide and N,N-dimethylacetoamide; urea solvents such as dimethylpropyleneurea; phosphoric triamide solvents such as hexamethylphosphoric triamide; ketone solvents such as acetone and methyl ethyl ketone; nitrile solvents such as acetonitrile and propionitrile may be used. Among them, water; aromatic hydrocarbon solvents such as benzene and toluene; ether solvents such as tetrahydrofuran, 1,4-dioxane and methyl tert-butyl ether; ester solvents such as ethyl acetate, isopropyl acetate and tert-butyl acetate are preferable, and toluene or ethyl acetate is more preferable. A solvent may be used by it self, or plural solvents may be mixed to be used. When mixed solvent is used, the mixing ratio is not limited.

The use amount of the solvent is preferably not more than 50 times by weight, and more preferably not more than 20 times by weight, relative to (S)-3-pyrrolidinol, since too much solvent is not preferable in terms of cost and post-processing.

The reaction temperature of the step 2 is preferably -10 to 90°C, and more preferably 0 to 50°C, for the purpose of the reduction of the reaction time and the inhibition of side reaction. The reaction time of the step 2 is not particularly limited and may be appropriately set; but, is preferably 1 to 24 hours, and more preferably 3 to 12 hours.

The method of adding the acylating reagent or carbamating reagent, base and reaction solvent to the reaction mixture of the step 1 and the addition order thereof are not particularly limited.

After the protection of the amino group is completed as the above, (S)-1-protected-3-pyrrolidinol represented by the compound (8) is extracted using an organic solvent for isolation.

Such an organic solvent is not limited as long as the water-solubility thereof is low, and exemplified by, for example, n-butanol, tetrahydrofuran, diethyl ether, ethylene glycol dimethyl ether, benzene, toluene, hexane, heptane, methylcyclohexane, carbon tetrachloride, chloroform, methylene chloride, 1,2-dichloroethane, chlorobenzene, ethyl acetate, isopropyl acetate and tert-butyl acetate, and is preferably toluene or ethyl acetate.

The use amount of the organic solvent is preferably not more than 50 times by weight, and more preferably not more than 20 times by weight, relative to the compound (8), since too much solvent is not preferable in terms of cost and post-processing.

The extract containing (S)-1-protected-3- pyrrolidinol represented by the formula (8) may be directly subject to the next step, or the target compound may be isolated by removing the solvent by a procedure such as heating under reduced pressure. Thus obtained target compound has high purity sufficient to be used in the subsequent step; however, may be further purified with general purification method such as crystallization, fractional distillation and column chromatography in order to further improve the yield of the subsequent steps and the purity of the compound obtained by the subsequent steps.

### Step 3

In the step 3, (S)-1-protected-3-(sulfonyloxy)-pyrrolidine represented by the following formula (2): 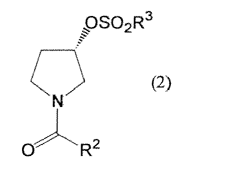 is produced by sulfonylating (S)-1-protected-3-pyrrolidinol represented by the formula (8).

In the formula, R² is the same as the above.

R³ is an optionally substituted alkyl group having 1 to 20 carbon atoms or an optionally substituted aryl group having 6 to 20 carbon atoms.

The alkyl group having 1 to 20 carbon atoms is exemplified by, for example, a methyl group, an ethyl group, an n-propyl group, an isopropyl group, an n-butyl group or a tert-butyl group. The aryl group having 6 to 20 carbon atoms is exemplified by, for example, a phenyl group, a naphthyl group or a biphenyl group.

The substituent is exemplified by, for example, halogen atoms such as a fluorine atom, a chlorine atom, a bromine atom and an iodine atom; a nitro group; a nitroso group; a cyano group; an amino group; a hydroxyamino group; an alkylamino group having 1 to 12 carbon atoms; an dialkylamino group having 1 to 12 carbon atoms; an aralkylamino group having 7 to 12 carbon atoms; a diaralkyl group having 7 to 12 carbon atoms; an alkylsulfonylamino group having 1 to 12 carbon atoms; a sulfonate group; a sulfonamide group; an azido group; a trifluoromethyl group; a carboxy group; an acyl group having 1 to 12 carbon atoms; an aroyl group having 7 to 12 carbon atoms; a hydroxy group; an alkyloxy group having 1 to 12 carbon atoms; an aralkyloxy group having 7 to 12 carbon atoms; an aryloxy group having 6 to 12 carbon atoms; an acyloxy group having 1 to 12 carbon atoms; an aroyloxy group having 7 to 12 carbon atoms; a silyloxy group having 3 to 12 carbon atoms; an alkylsulfonyloxy group having 1 to 12 carbon atoms; or an alkylthio group having 1 to 12 carbon atoms.

R³ is preferably a methyl group, a trifluoromethyl group, a phenyl group, a 4-methylphenyl group, a 4-chlorophenyl group, a 2-nitrophenyl group, a 3-nitrophenyl group, a 4-nitrophenyl group, and more preferably a methyl group.

The sulfonylation can be carried out with a sulfonylating reagent in the presence of a base.

The sulfonylating reagent is exemplified by methanesulfonyl chloride, trifluoromethanesulfonyl anhydride, benzenesulfonyl chloride, 4-methylbenzenesulfonyl chloride, 4-chlorobenzenesulfonyl chloride, 2-nitrobenzenesulfonyl chloride, 3-nitrobenzenesulfonyl chloride, 4-nitrobenzenesulfonyl chloride, and is more preferably methanesulfonyl chloride.

The use amount of the sulfonylating reagent is preferably 2 to 10 times by mole, and more preferably 2 to 5 times by mole, relative to the compound (8).

The base used in the step 3 is exemplified by tertiary amines such as triethylamine, tri-n-butylamine, N-methylmorpholine, N-methylpiperidine, diisopropylethylamine, pyridine, N,N-dimethylaminopyridine and 1,4-diazabicyclo[2,2,2]octane; metal hydroxides such as lithium hydroxide, sodium hydroxide, potassium hydroxide, barium hydroxide and magnesium hydroxide; metal carbonates such as lithium carbonate, sodium carbonate and potassium carbonate; metal hydrogencarbonates such as lithium hydrogencarbonate, sodium hydrogencarbonate and potassium hydrogencarbonate.

The base is preferably tertiary amines such as triethylamine, tri-n-butylamine, N-methylmorpholine, N-methylpiperidine, diisopropylethylamine, pyridine, N,N-dimethylaminopyridine and 1,4-diazabicyclo[2,2,2]octane, and more preferably triethylamine.

The use amount of the base is preferably 2 to 20 times by mole, and more preferably 2 to 10 times by mole, relative to the compound (8).

The base may be used as a reaction solvent in the stop 3; however, a reaction solvent may be further added to ensure flow property when only minimum requisite base is added in terms of economic efficiency.

As such a reaction solvent, for example, water; ether solvents such as tetrahydrofuran, diethyl ether, 1,4-dioxane and ethylene glycol dimethyl ether; aromatic hydrocarbon solvents such as benzene and toluene; aliphatic hydrocarbon solvents such as pentane, hexane, heptane and methylcyclohexane; halogenated solvents such as carbon tetrachloride, chloroform, methylene chloride, 1,2-dichloroethaneandchlorobenzene; ester solvents such as ethyl acetate, isopropyl acetate and tert-butyl acetate; sulfoxide solvents such as dimethylsulfoxide; amide solvents such as N,N-dimethylformamide and N,N-dimethylacetoamide; urea solvents such as dimethylpropyleneurea; phosphoric triamide solvents such as hexamethylphosphoric triamide; ketone solvents such as acetone and methyl ethyl ketone; nitrile solvents such as acetonitrile and propionitrile may be used.

The solvent is preferably ether solvents such as tetrahydrofuran, diethyl ether, 1,4-dioxane and ethylene glycol dimethyl ether; aromatic hydrocarbon solvents such as benzene and toluene; ester solvents such as ethyl acetate, isopropyl acetate and tert-butyl acetate, and more preferably toluene or ethyl acetate. A solvent may be used by it self, or plural solvents may be used in combination. When plural solvents may be used in combination, the mixing ratio is not particularly limited.

The use amount of the reaction solvent is preferably not more than 50 times by weight, and more preferably not more 20 times by weight, relative to the compound (8), since too much solvent is not preferable in terms of cost and post-processing.

The reaction temperature of the step 3 is preferably -40 to 80°C, and more preferably -20 to 50°C, for the purpose of the reduction of the reaction time and the inhibition of side reaction.

The reaction time of the step 3 is not particularly limited, and is preferably 0.1 to 24 hours, and more preferably 0.5 to 12 hours.

The order and method of adding the compound (8), base, sulfonylating reagent and reaction solvent in the step 3 are not particularly limited.

As the procedure after the reaction, a general procedure for obtaining a product from a reaction mixture may be carried out. For example, water, or if needed, an acid aqueous solution such as hydrochloric acid and sulfuric acid or an alkaline aqueous solution such as sodium hydroxide aqueous solution and potassium carbonate aqueous solution for neutralization is added to the reaction mixture, and extraction is carried out using a general extraction solvent such as ethyl acetate, diethyl ether, methylene chloride, toluene and hexane. The reaction solvent and extraction solvent are distilled away from the obtained extraction mixture by heating under reduced pressure to obtain the target compound. Thus obtained target compound has the purity sufficient to be used for the subsequent step; however, may be further purified with a general purification method such as crystallization, fractional distillation and column chromatography for further improving the yield of the subsequent steps and the purity of the compound obtained by the subsequent steps.

### Step 4

In the step 4, (S)-1-protected-3-(1-cyano-1,1-diphenylmethyl)-pyrrolidine represented by the following formula (4): 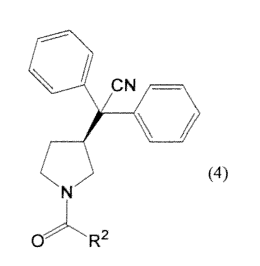 is produced by reacting (S)-1-p-rotected-3-(sulfonyloxy)-pyrrolidine represented by the formula (2) with diphenylacetonitrile represented by the following formula (3): 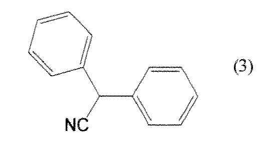 in the presence of a base.

In the formula, R² is the same as the above.

The use amount of diphenylacetonitrile represented by the formula (3) is preferably 1 to 10 times by mole, and more preferably 1 to 3 times by mole, relative to the compound (2).

The base is exemplified by, for example, metal amides such as lithium amide, sodium amide, lithium diisopropylamide, sodium hexamethyldisilazide, potassium hexamethyldisilazide and magnesium diisopropylamide chloride; organic lithium reagents such as methyllithium, n-butyllithium, tert-butyllithium, sec-butyllithium and phenyllithium; Grignard reagents such as methylmagnesium bromide, n-butylmagnesium chloride, sec-butylmagnesium chloride and tert-butylmagnesium chloride; metal alkoxides such as lithium methoxide, sodium methoxide, potassium methoxide, lithium ethoxide, sodium ethoxide, potassium ethoxide, magnesium ethoxide, sodium isopropoxide, potassium isopropoxide, lithium tert-butoxide, sodium tert-butoxide and potassium tert-butoxide; metal hydrides such as lithium hydride, sodium hydride, potassium hydride and calcium hydride; metal carbonates such as lithium carbonate, sodium carbonate, potassium carbonate, sodium carbonate and calcium carbonate; metal hydrogencarbonates such as lithium hydrogencarbonate, sodium hydrogencarbonate, potassium hydrogencarbonate and cesium hydrogencarbonate; metal hydroxides such as lithium hydroxide, sodium hydroxide and potassium hydroxide; tertiary amines such as triethylamine, ethyldiisopropylamine and pyridine.

The base is preferablymetal alkoxides such as lithium methoxide, sodium methoxide, potassium methoxide, lithium ethoxide, sodium ethoxide, potassium ethoxide, magnesium ethoxide, sodium isopropoxide, potassium isopropoxide, lithium tert-butoxide, sodium tert-butoxide and potassium tert-butoxide; metal hydrides such as lithium hydride, sodium hydride, potassium hydride and calcium hydride, and more preferably sodium hydride, sodium tert-butoxide or potassium tert-butoxide.

The use amount of the base is preferably 0.5 to 20 times by mole, and more preferably 1 to 5 times by mole, relative to the compound (2).

In the step 4, a reaction solvent may be further added to ensure flow property. As such a solvent, ether solvents such as tetrahydrofuran, diethyl ether, 1,4-dioxane, ethylene glycol dimethyl ether; aromatic hydrocarbon solvents such as benzene and toluene; aliphatic hydrocarbon solvents such as pentane, hexane, heptane and methylcyclohexane; halogenated solvents such as carbon tetrachloride, chloroform, methylene chloride, 1,2-dichloroethane and chlorobenzene; ester solvents such as ethyl acetate, isopropyl acetate and tert-butyl acetate; sulfoxide solvents such as dimethylsulfoxide; amide solvents such as N,N-dimethylformamide and N,N-dimethylacetoamide; urea solvents such as dimethylpropyleneurea; phosphoric triamide solvents such as hexamethylphosphoric triamide; ketone solvents such as acetone and methyl ethyl ketone; nitrile solvents such as acetonitrile and propionitrile may be used.

The solvent is preferably ether solvents such as tetrahydrofuran, diethyl ether, 1,4-dioxane and ethylene glycol dimethyl ether; aromatic hydrocarbon solvents such as benzene and toluene; ester solvents such as ethyl acetate, isopropyl acetate and tert-butyl acetate; amide solvents such as N,N-dimethylformamide and N,N-dimethylacetoamide, and more preferably tetrahydrofuran, toluene, ethyl acetate or N,N-dimethylacetoamzde. A solvent may be used by itself, or plural solvents may be mixed to be used. When mixed solvent is used, the mixing ratio is not limited.

The use amount of the solvent is preferably not more than 50 times by weight, and more preferably not more 20 times by weight, relative to the compound (2), since too much solvent is not preferable in terms of cost and post-processing.

The reaction temperature of the step 4 is preferably -40 to 80°C, and more preferably -20 to 50°C, for the purpose of the reduction of the reaction time and the inhibition of side reaction.

The reaction time of the step 4 is not particularly limited, and is preferably 0.1 to 24 hours, and preferably 0.5 to 12 hours.

The order and method of adding the compound (2), the compound (3), base and reaction solvent in the step 4 are not particularly limited.

As the procedure after the reaction, a general procedure for obtaining a product from a reaction mixture may be carried out. For example, water, or if needed, an acid aqueous solution such as hydrochloric acid and sulfuric acid for neutralization is added to the reaction mixture, and extraction is carried out using a general extraction solvent such as ethyl acetate, diethyl ether, methylene chloride, toluene and hexane. The reaction solvent and extraction solvent are distilled away from the obtained extraction mixture by heating under reduced pressure to obtain the target compound. Thus obtained target compound has the purity sufficient to be used for the subsequent step; however, may be further purified with a general purification method such as crystallization, fractional distillation and column chromatography for further improving the yield of the subsequent steps and the purity the compound obtained by the subsequent steps.

The (S)-1-protected-3-(1-cyano-1,1-diphenylmethyl)-pyrrolidine which is represented by the following formula (1): 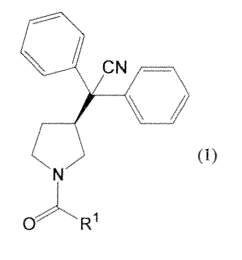 wherein, R¹ is a methyl group, a phenyl group, a tert-butoxy group or a benzyloxy group,
and produced by the step 4 is a novel compound, which has not been described in any documents.

### Step 5

In the step 5, (S)-3-(1-cyano-1,1-diphenylmethyl)-pyrrolidine or a salt thereof is produced by deprotecting (S)-1-protected-3-(1-cyano-1,1-diphenylmethyl)-pyrrolidine represented by the formula (4). The (S)-3-(1-cyano-1,1-diphenylmethyl)-pyrrolidine is represented by the following formula (5).

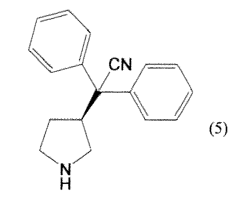

The condition for deprotection in the step 5 may be appropriately set depending on the kind of the protective group. For example, the condition may be set according to the method of cleavaging an acyl group or carbamate group, which method is described in pages 503 to 564 of JOHN WILEY & SONS, INC., Theodora W. Greene, PROTECTIVE GROUPS in ORGANIC SYNTHESIS Third edition.

Specifically, a formyl group and trifluoroacetyl group can be cleavaged by alkali hydrolysis. In addition, a benzyloxycarbonyl group can be cleavaged by hydrogenaration in the presence of a metal catalyst such as palladium carbon. Furthermore, an allyloxycarbonayl group can be cleavaged by hydrolysis in the presence of a metal catalyst such as ruthenium.

Both of an acyl group and carbamate group are preferably cleavaged by acidic hydrolysis or alcohol degradation by acidic alcoholysis. The acid is exemplified by inorganic acids such as hydrochloride, hydrobromide, perchloric acid and sulfuric acid; organic acids such as formic acid, methanesulfonic acid and p-toluenesulfonic acid. The use amount of the acid is preferably 0.5 to 30 times by mole, and more preferably 1 to 10 times by mole, relative to the compound (4). The alcohol is exemplified by methanol, ethanol, n-propanol, isopropanol, n-butanol and ethylene glycol. The use amount of the above water or alcohol is preferably not more than 50 times by weight, and more preferably not more than 20 times by weight, relative to the compound (4). The alcohol may be mixed with water, and the mixing ratio thereof is not limited.

The reaction temperature and time of the step 5 may be appropriately set depending on the kind of the protective group. For example, a tert-butoxycarbonyl group can be cleavaged for about 1 to 12 hours at 0 to 50°C. In addition, a benzoyl group can be cleavaged for about 12 to 36 hours at 50 to 100°C.

In the step 5, the order and method of adding the compound (4), acid and water or alcohol are not particularly limited.

As the procedure after the reaction, a general procedure for obtaining a product from a reaction mixture may be carried out. For example, water, or if needed, an alkaline aqueous solution such as sodium hydroxide aqueous solution and potassium carbonate aqueous solution for neutralization is added to the reaction mixture, and extraction is carried out using a general extraction solvent such as ethyl acetate, diethyl ether, methylene chloride, toluene and hexane. The reaction solvent and extraction solvent are distilled away from the obtained extraction mixture by heating under reduced pressure to obtain the target compound. Thus obtained target compound has the purity sufficient to be used for the subsequent step; however, may be further purified with a general purification method such as crystallization, fractional distillation and column chromatography for further improving the yield of the subsequent steps and the purity the compound obtained by the subsequent steps.

The compound (5) may be crystallized to be isolated as a salt with the acid by distilling the solvent from the reaction mixture after the reaction.

In the step 5, when the compound (4) is deprotected, (S)-3-(1-carboxyamide-1,1-diphenylmethyl)-pyrrolidine represented by the following formula (9): 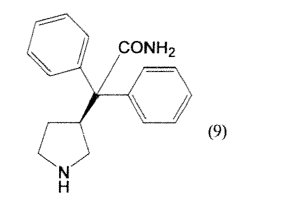 is possibly produced in some cases by hydration of the cyano group of the compound (4) at the same time as the deprotection or in a serial manner. Such a case is included in the scope of the present invention.

### EXAMPLES

Hereinafter, the present invention is described in more detail with examples; however, it is not intended that the present invention be limited to the examples.

The purity of (S)-pyrrolidinol derivatives and (S)-pyrrolidine derivatives in the examples was analyzed with the following HPLC method. The "arena%" as purity means chemical purity represented as dimensional percentage, and "wt%" means content amount (% by weight).

### Chemical purity analysis method and Content amount analysis method (Gradient method)

Column: COSMOSIL 5C18ARII, manufactured by Nacalai, 250 × 4.6 mm
Mobile phase A: 0.1% phosphoric acid aqueous solution
Mobile phase B: acetonitrile
Mobile phase A (%) / Mobile phase B (%) = 90 / 10 to 40 / 60
Flow rate: 1.0 ml/min
Detection: UV 210nm
Column temperature: 40°C
Retention time:
   (S)-1-(tert-butoxycarbonyl)-3-pyrrolidinol 11.8 min,
   (S)-1-(tert-butoxycarbonyl)-3-(methanesulfonyloxy)-pyrrolidine 14.4 min,
   (S)-1-(tert-butoxycarbonyl)-3-(1-cyano-1,1-diphenylmethyl)-pyrrolidine 30.5 min
   (S)-3-(1-cyano-1,1-diphenylmethyl)-pyrrolidine 10.3 min,
   (S)-1-benzoyl-3-(1-cyano-1,1-diphenylmethyl)-pyrrolidine 19.8 min

### Optical purity analysis method (Isocratic method)

The measurement was carried out after the amino group of 3-(1-cyano-1,1-diphenylmethyl)-pyrrolidine was protected with tert-butoxycarbonyl, which is hereinafter represented as "Boc".
Column: CHIPALCEL OD-H, produced by Daicel chemical industries, Ltd., 250 × 4.6 mm
Mobile phase: hexane / isopropanol = 98 / 2 by volume
Flow rate: 1.0 ml/min
Detection: UV 210 nm
Column temperature: 30°C
Retention time:
   (R)-3-(1-cyano-1,1-diphenylmethyl)-pyrrolidine protected with Boc 20.3 min,
   (S)-3-(1-cyano-1,1-diphenylmethyl)-pyrrolidine protected with Boc 22.3 min

### Example 1: Production of (S)-1-(tert-butoxycarbonyl)-3-pyrrolidinol

(S)-4-Chloro-3-hydroxybutyronitrile (2.41g) was dissolved in methanol (12.4 ml), and concentrated hydrochloric acid (2.51 g) and 10% palladium carbon containing 50wt% of water (0.48 g) were added thereto. Under pressured hydrogen at 4 atm, the mixture was stirred at 25°C for 23 hours and at 50°C for 6 hours. Then, 5% palladium carbon containing 50wt% of water (0.48 g) was added thereto, and the mixture was stirred at 50°C for 11 hours.

The catalyst was removed with filtration under reduced pressure, and the obtained filtrate was concentrated under reduced pressure. To the concentrate, methanol (6.4 ml) and distilled water (1.6 ml) and triethylamine (2.29 g) were added, and the mixture was stirred at 25°C for 20 hours and at 40°C for 8 hours.

To the reaction mixture, di-tert-butyldicarbonate (4.82 g) and triethylamine (2.10 g) were added, and the mixture was stirred at 25°C for 2 hours. The reaction mixture was concentrated to 9.92 g under reduced pressure. To the concentrate, toluene (20 ml) and distilled water (3.5 ml) were added in order to extract the target compound into the organic layer. The obtained organic layer was washed with distilled water 1 ml) and concentrated under reduced pressure to obtain 3.94 g of the target compound as a brown oil (content amount: 67.5.wt%, yield: 71%).

¹H-NMR (CDCl₃): δ (ppm) 1.48(s, 9H), 1.86-2.04(m, 3H), 3.26-3.56(m, 4H), 4.45(m, 1H)

### Example 2: Production of (S)-1-(tert-butoxycarbonyl)-3-(methanesulfonyloxy)pyrrolidine

The (S)-1-(tert-butoxycarbonyl)-3-pyrrolidinol (3.61 g) produced in Example 1 was dissolved in toluene (30 ml), and triethylamine (1.58 g) was added thereto. To the solution, methanesulfonyl chloride (1.64 g) was added dropwise on an ice bath over 10 minutes, and the mixture was stirred at 25°C for 1 hour. To the reaction mixture, distilled water (15ml) was added in order to extract the target compound into the organic layer. The obtained organic layer was washed with distilled water (15 ml). The organic layer was concentrated under reduced pressure to obtain 4.10 g of the target compound as a brown oil (content amount: 74.6wt%, yield: 89%).

¹H-NMR (CDCl₃): δ (ppm) 1.47(s, 9H), 2.15(m, 1H), 2.27(m, 1H), 3.05(s, 3H), 3.42-3.78(m, 4H), 5.26(m, 1H)

### Example 3: Production of (S)-1-(tert-butoxycarbonyl)-3-(1-cyano-1,1-diphenylmethyl)-pyrrolidine

The toluene solution (2.23 g) containing (S)-1-(tert-butoxycarbonyl)-3-(methanesulfonyloxy)pyrrolidine (1.56 g) was concentrated under reduced pressure. To the concentrate, tetrahydrofuran (1.51 g) and potassium tert-butoxide (0.79g) were added. To the mixture, the solution consisting of diphenylacetonitrile (1.37 g) and tetrahydrofuran (1.63 g) was added dropwise over 1 hour under reflux condition. The mixture was stirred for 21 hours. After the mixture was cooled to room temperature, distilledwater (2.8 ml) and toluene (5 ml) were added thereto in order to extract the target compound into the organic layer. The obtained organic layer was washed with distilled water (2.5 ml) twice. The organic layer was concentrated under reduced pressure and dried in vacuo to obtain 2.47 g of the target compound as an orange oil (content amount: 77.7wt%, yield: 90%).

The obtained target compound was purified with column chromatography (packing material: silica gel, eluent: ethyl acetate / hexane = 1 / 2), and the obtained fraction was concentrated under reduced pressure and dried in vacuo to obtain 1.57 g of the target compound as a white solid (content amount: 97.4wt%, optical purity: 100%ee, yield: 72%).

¹H-NMR (CDCl₃): δ (ppm) 1.44(s, 9H), 1.78-2.14(m, 2H), 3.25-3.40(m, 3H), 3.42-3.68(m, 2H), 7.24-7.42(m, 6H), 7.42-7.60(m, 4H)

### Example 4: Production of hydrobromic acid salt of (S)-3-(1-cyano-1,1-diphenylmethyl)-pyrrolidine

(S)-1-(tert-butoxycarbonyl)-3-(1-cyano-1,1-diphenylmethyl)-pyrrolidine (1.13 g) was dissolved in tetrahydrofuran (5.62 g), and 47% hydrobromic acid (1.55 g) was added thereto. The solution was stirred at 40°C for 6 hours. The solution was cooled to room temperature, and then concentrated to 2.42 g under reduced pressure. To the concentrate, toluene (0.42 g) and ethyl acetate (2.39 g) were added. The mixture was concentrated to 3.15 g under reduced pressure, and seed crystal was added thereto to precipitate the target compound as crystals. After the mixture was stirred at 20°C for 5 minutes, toluene (1.49 g) and ethyl acetate (4.02 g) were added thereto. The mixture was further stirred at 20°C for 1 hour. The crystals were filtrated under reduced pressure. The crystals were washed with ethyl acetate (2 ml) and dried in vacuo to obtain 0.26 g of the target compound as white crystals (chemical purity: 99.9area%, optical purity: 100%ee, yield: 26%).

The separated filtrate was concentrated under reduced pressure, and the target compound was further precipitated as crystals. Ethyl acetate (4.02g) was added thereto, and the mixture was stirred at 20°C for 30 minutes. The crystals were filtrated under reduced pressure, and washed with ethyl acetate (5 ml). Then the crystals were dried in vacuo to obtain 0.39 g of the target compound as white crystals (chemical purity: 99.9area%, optical purity: 100%ee, yield: 38%).

¹H-NMR (D₂O): δ(ppm) 2.03(m, 1H), 2.23(m, 1H), 3.22(dd, 1H), 3.40(m, 1H), 3.53-3.59(m, 2H), 3.95(m, 1H), 7.43(t, 2H), 7.47(t, 4H), 7.59(d, 4H)

### Example 5: Production of (S)-1-benzoyl-3-(1-cyano-1,1-diphenylmethyl)-pyrrolidine

To (S)-1-benzoyl-3-(methanesulfonyloxy)pyrrolidine (0.81g), tetrahydrofuran (1.51 g) and potassium tert-butoxide (0.40 g) were added. The solution consisting of diphenylacetonitrile (1.37 g) and tetrahydrofuran (1.63 g) was added thereto dropwise over 1 hour under reflux condition, and the mixture was stirred for 21 hours. The reaction mixture was cooled to room temperature, and distilled water (2.8 ml) and toluene (5 ml) were added thereto in order to extract the target compound into the organic layer. The obtained organic layer was washed with distilled water (2.5 ml) twice. The organic layer was concentrated under reduced pressure, and dried in vacuo to obtain 1.19 g of the target compound as a white solid (content amount: 80.0wt%, yield: 86%).

¹H-NMR (CDCl₃): δ(ppm) 1.96(m, 1H), 2.08(m, 1H), 3.34-3.72(m, 4H), 3.89(m, 1H), 7.25-7.54(m, 15H)

### Example 6: Production of a hydrobromic acid salt of (S)-3-(1-cyano-1,1-diphenylmethyl)-pyrrolidine

To (S)-1-benzoyl-3-(1-cyano-1,1-diphenylmethyl)-pyrrolidine (171 mg), 47% hydrobromic acid (3.22 g) was added, and the mixture was stirred at 110°C to 120°C for 38 hours. The mixture was cooled to room temperature, and concentrated under reduced pressure. To the concentrate, distilled water (5 ml) and ethyl acetate (5 ml) were added in order to extract the target compound to the water layer (yield: 66%). Isopropanol (5 ml) was added to the water layer, and the mixture was concentrated under reduced pressure to precipitate crystals. Isopropanol (5ml) was further added thereto, and the mixture was concentrated under reduced pressure. Ethyl acetate (1ml) was further added thereto, and the mixture was stirred at 20°C for 30 minutes. The crystals were separated by filtration under reduced pressure, and washed with ethyl acetate (2 ml). The crystals were dried in vacuo to obtain 113 mg of the target compound as white crystals (content amount: 59.8wt%, yield: 53%).

¹H-NMR (D₂O): δ(ppm) 2.03(m, 1H), 2.23(m, 1H), 3.22(dd, 1H), 3.40(m, 1H), 3.53-3.59(m, 2H), 3.95(m, 1H), 7.43(t, 2H), 7.47(t, 4H), 7.59(d, 4H)

### Example 7: Production of (S)-3-(1-cyano-1,1-diphenylmethyl)-pyrrolidine

To a hydrobromic acid salt of (S)-3-(1-cyano-1,1-diphenylmethyl)-pyrrolidine (78 mg, content amount: 60.8wt%), distilled water (1 ml) and toluene (1 ml) and 30wt% aqueous solution of sodium hydroxide (38 mg) were added. The mixture was stirred, and the target compound was extracted into the organic layer. The obtained organic layer was washed with distilled water (1 ml) twice, and concentrated under reduced pressure. The residue was dried in vacuo to obtain 32 mg of the target compound as a colorless transparent oil (content amount: 100wt%, yield: 88%).

¹H-NMR (CDCl₃): δ(ppm) 1.74(m, 1H), 1.92(m, 1H), 2.04(br, 1H), 2.88-2.95(m, 2H), 3.00(dd, 1H), 3.13(m, 1H), 3.30(m, 1H), 7.27(m, 2H), 7.33(m, 4H), 7.45(m, 4H)

## Claims

1. An (S)-1-protected-3-(1-cyano-1,1-diphenylmethyl)-pyrrolidine represented by the following formula (1): 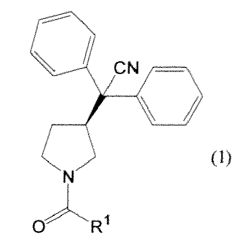 wherein, R¹ is a methyl group, a phenyl group, a tert-butoxy group or a benzyloxy group.

2. An (S)-1-(tert-butoxycarbonyl)-3-(1-cyano-1,1-diphenylmethyl)-pyrrolidine, wherein R² is a tert-butoxy group in the formula (1).

3. A method of producing an (S)-1-protected-3-(1-cyano-1,1-diphenylmethyl)-pyrrolidine,
wherein the (S)-1-protected-3-(1-cyano-1,1-diphenylmethyl)-pyrrolidine is represented by the following formula (4): 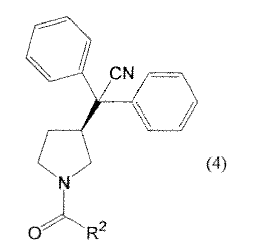 wherein, R² is a hydrogen atom, an optionally substituted alkyl group having 1 to 20 carbon atoms, an optionally substituted alkenyl group having 2 to 20 carbon atoms, an optionally substituted aralkyl group having 7 to 20 carbon atoms, an optionally substituted aryl group having 6 to 20 carbon atoms, an optionally substituted alkyloxy group having 1 to 20 carbon atoms, an optionally substituted alkenyloxy group having 2 to 20 carbon atoms, an optionally substituted aralkyloxy group having 7 to 20 carbon atoms, or an optionally substituted aryloxy group having 6 to 20 carbon atoms,
comprising a step of reacting an (S)-1-protected-3-(sulfonyloxy)-pyrrolidine represented by the following formula (2): 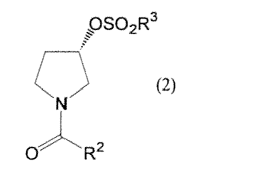 wherein, R² is the same as the above; R³ is an optionally substituted alkyl group having 1 to 20 carbon atoms, or an optionally substituted aryl group having 6 to 20 carbon atoms,
with diphenylacetonitrile represented by the following formula (3) : 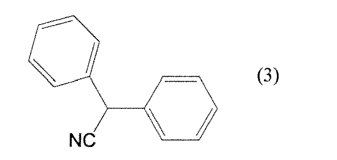 in the presence of a base.

4. A method of producing (S)-3-(1-cyano-1,1-diphenylmethyl)-pyrrolidine or a salt thereof,
wherein the (S)-3-(1-cyano-1,1-diphenylmethyl)-pyrrolidine is represented by the following formula (5): 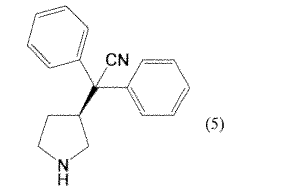 comprising a step of deprotecting the (S)-1-protected-3-(1-cyano-1,1-diphenylmethyl)-pyrrolidine represented by the formula (4) and produced by the method according to claim 3.

5. A method of producing an (S)-1-protected-3-pyrrolidinol,
wherein the (S)-1-protected-3-pyrrolidinol is represented by the following formula (8): 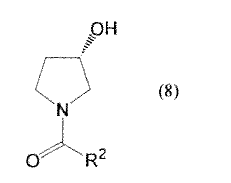 wherein R² is a hydrogen atom, an optionally substituted alkyl group having 1 to 20 carbon atoms, an optionally substituted alkenyl group having 2 to 20 carbon atoms, an optionally substituted aralkyl group having 7 to 20 carbon atoms, an optionally substituted aryl group having 6 to 20 carbon atoms, an optionally substituted alkyloxy group having 1 to 20 carbon atoms, an optionally substituted alkenyloxy group having 2 to 20 carbon atoms, an optionally substituted aralkyloxy group having 7 to 20 carbon atoms, or an optionally substituted aryloxy group having 6 to 20 carbon atoms,
comprising steps of reducing (S)-4-chloro-3-hydroxybutyronitrile represented by the following formula (6): 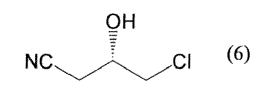 to obtain (S)-3-pyrrolidinol represented by the following formula (7): 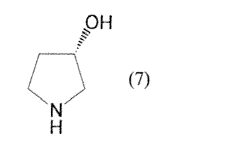 and then protecting the amino group without isolating the compound (7).

6. A method of producing an (S)-1-protected-3-(sulfonyloxy)-pyrrolidine,
wherein the (S)-1-protected-3-(sulfonyloxy)-pyrrolidine is represented by the following formula (2): 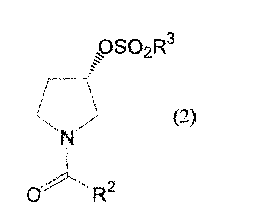 wherein, R² is a hydrogen atom, an optionally substituted alkyl group having 1 to 20 carbon atoms, an optionally substituted alkenyl group having 2 to 20 carbon atoms, an optionally substituted aralkyl group having 7 to 20 carbon atoms, an optionally substituted aryl group having 6 to 20 carbon atoms, an optionally substituted alkyloxy group having 1 to 20 carbon atoms, an optionally substituted alkenyloxy group having 2 to 20 carbon atoms, an optionally substituted aralkyloxy group having 7 to 20 carbon atoms, or an optionally substituted aryloxy group having 6 to 20 carbon atoms; R³ is an optionally substituted alkyl group having 1 to 20 carbon atoms, or an optionally substituted aryl group having 6 to 20 carbon atoms,
comprising a step of sulfonylating the (S)-1-protected-3-pyrrolidinol represented by the formula (8) and produced by the method according to claim 5.

7. A method of producing an (S)-1-protected-3-(1-cyano-1,1-diphenylmethyl)-pyrrolidine,
wherein the (S)-1-protected-3-(1-cyano-1,1-diphenylmethyl)-pyrrolidine is represented by the following formula (4): 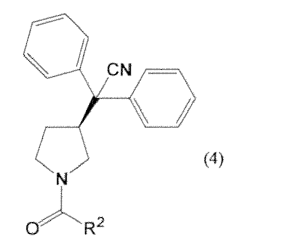 wherein, R² is a hydrogen atom, an optionally substituted alkyl group having 1 to 20 carbon atoms, an optionally substituted alkenyl group having 2 to 20 carbon atoms, an optionally substituted aralkyl group having 7 to 20 carbon atoms, an optionally substituted aryl group having 6 to 20 carbon atoms, an optionally substituted alkyloxy group having 1 to 20 carbon atoms, an optionally substituted alkenyloxy group having 2 to 20 carbon atoms, an optionally substituted aralkyloxy group having 7 to 20 carbon atoms, or an optionally substituted aryloxy group having 6 to 20 carbon atoms,
comprising a step of reacting the (S)-1-protected-3-(sulfonyloxy)-pyrrolidine represented by the formula (2) and produced by the method according to claim 6 with diphenylacetonitrile represented by the following formula (3): 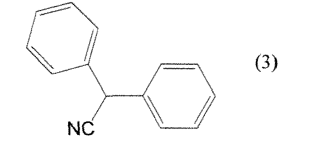 in the presence of a base.

8. A method of producing (S)-3-(1-cyano-1,1-diphenylmethyl)-pyrrolidine or a salt thereof,
wherein the (S)-3-(1-cyano-1,1-diphenylmethyl)-pyrrolidine is represented by the following formula (5): 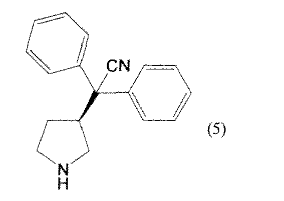 comprising a step of deprotecting the (S)-1-protected-3-(1-cyano-1,1-diphenylmethyl)-pyrrolidine represented by the formula (4) and produced by the method according to claim 7.
